# FASCICULE DE BREVET EUROPEEN

(11) **EP 0 362 094 B1**
(45) Date de publication et mention de la délivrance du brevet: **10.02.1993**
(21) Numéro de dépôt: 89440091.0
(22) Date de dépôt: 01.09.1989
(51) Int. Cl.: A61F 13/15, A61F 13/58

(54) **Couche-culotte**
Windel
Napkin

(30) Priorité: 28.09.1988 FR 8813061
(43) Date de publication de la demande: 04.04.1990
(73) Titulaire: CELATOSE Société anonyme dite:, F-59200 Tourcoing (Nord) (FR)
(72) Inventeur: Bruneau, Jean-Pierre, F-59310 Orchies (Nord) (FR)
(74) Mandataire: Rodhain, Claude

(56) Documents cités:
- EP-A- 0 211 197
- FR-A- 2 515 004
- GB-A- 2 185 176
- US-A- 3 610 244
- US-A- 4 725 468

## Description

L'invention est relative à une couche culotte qui trouvera notamment son application dans le domaine de l'hygiène infantile et/ou médicale.

Depuis de nombreuses années, on connait les couches culottes jetables. Leur aspect pratique et l'absence d'entretien ont largement contribué au développement de celles-ci qui ont d'ailleurs fait l'objet de nombreux perfectionnements.

On connait de telles couches culottes formées d'une feuille extérieure imperméable, notamment étanche aux liquides, généralement constituée par un film de polyéthylène et d'une feuille intérieure perméable de confort, généralement en voile de non tissé, entre lesquelles est disposé un matelas formé de matériau absorbant (voir US-A- 3610244). A ce sujet, des efforts particuliers ont été mis en oeuvre pour augmenter, d'une part l'absorption de la couche culotte et d'autre part augmenter l'étanchéité. Le pouvoir d'absorption plus important permet notamment avec moins de matière de retenir une quantité de liquide importante. Toutefois, de ce fait, il est nécessaire que les couches présentent une certaine étanchéité pour éviter le rejet, vers l'extérieur de la couche, du liquide absorbé. Plus précisément, il a été tout d'abord nécessaire de renforcer l'étanchéité au niveau de l'entrejambe du porteur puis également de renforcer l'étanchéité au niveau de la ceinture de la couche.

A ce sujet, on connait des dispositifs "anti-fuite" au niveau de l'entrejambe, formés par différents systèmes élastiques d'entrejambe qui permettent d'adapter la forme de la couche à la morphologie du porteur.

Par ailleurs, au niveau de la ceinture, une première amélioration a été remarquée grâce à des dispositifs de fermeture adhésifs qui permettent de former une ceinture adaptable au mieux par rapport aux mensurations du porteur.

Ainsi, on cherche à éviter l'entrebaillement de la couche qui, selon la position du porteur, peut être néfaste en permettant des fuites lors d'un apport important instantané en liquide du fait de l'inertie du matériau absorbant.

Pour compléter l'étanchéité au niveau de la ceinture, on a également développé des systèmes de ceinture élastique qui permettent de rendre la taille extensible devant ou derrière et autorisent la variation dans le temps de la morphologie du porteur. En effet, dans le cas d'un bébé par exemple, les variations de la taille entre les positions couchée et debout ou encore entre deux repas sont très sensibles; la ceinture élastique permet alors de pallier ces variations sans être obligé de relanger le bébé.

A ce sujet, on connait notamment une première réalisation dans laquelle est placé un matériau élastique traditionnel ou une bande de mousse élastique, telle qu'une mousse de polyuréthane dont la dimension est légèrement inférieure à la largeur des parties arrière et/ou avant de la couche.

On connait également une autre réalisation qui prévoit au niveau de la partie avant et/ou de la partie arrière un ruban thermorétractile, sensible à la chaleur, pour provoquer sa rétractation et provoquer l'élasticité requise.

Ces différentes réalisations de couche culotte avec ceinture élastique ont donc pour but d'éviter certaines fuites en adaptant la ceinture de la couche à la taille du porteur afin d'augmenter le contact et d'éviter les entrebaillements néfastes.

Cependant, afin que l'efficacité du système à la ceinture soit importante, on a constaté qu'il était nécessaire de placer les dispositifs de fermeture adhésifs dans le prolongement de la ceinture élastique.

A ce sujet, on connait de telles réalisations dans lesquelles la ceinture est réalisée par exemple par une bande de matériau élastique dans le prolongement de laquelle sont disposés des dispositifs d'attache rapportés et placés bout à bout par rapport à la bande.

Toutefois, si de tels systèmes sont plus efficaces au niveau de l'étanchéité, il est constant de constater des déchirements des dispositifs d'attache car les contraintes sont localisées au niveau de la zone de fixation des dispositifs d'attache.

Le but de la présente invention est de proposer une couche, qui trouvera notamment son application dans le domaine de l'hygiène infantile et/ou médicale, qui permet de pallier les inconvénients des réalisations connues en proposant une couche plus performante sur le plan de l'élastification de la taille, de la fermeture, de la répétitivité de la fermeture et de l'étanchéité au niveau de la ceinture.

Un des buts de la présente invention est de proposer une couche culotte présentant une ceinture élastique et des dispositifs de fixation disposés dans le prolongement et qui coopèrent ensemble pour répartir les contraintes de traction tout au long de la ceinture.

Ainsi, contrairement aux couches connues dans lesquelles les contraintes sont localisées au niveau des attaches et dans lesquelles on constate constamment des arrachements de la feuille extérieure imperméable au niveau des dits attaches, selon la présente invention, la répartition des contraintes tout le long de la ceinture permet d'éviter les arrachements intempestifs du film extérieur de la couche.

Par ailleurs, l'effort de tension au niveau de la ceinture est parfaitement réparti entre les deux attaches ce qui permet à la ceinture, notamment sur la cambrure du dos et le ventre du porteur, d'épouser parfaitement la morphologie du porteur.

Un autre but de la présente invention est de proposer une couche culotte dont la ceinture élastique est le plus proche possible des extrémités de la couche pour éviter "l'effet de bavette" procuré par les couches traditionnelles.

En effet, généralement la ceinture élastique est décalée par rapport aux extrémités de la couche de la sorte il subsiste à la partie arrière et à la partie avant de la couche une zone, couramment appelée "bavette", qui est malheureusement repliée par l'utilisateur vers l'intérieur de la couche, ce qui est néfaste pour l'étanchéité car on réduit l'efficacité de la ceinture élastique.

Grâce à la structure de la réalisation de la ceinture élastique de la présente invention, cette zone peut être minimisée, voire même supprimée.

D'autres buts et avantages de la présente invention apparaitront au cours de la description qui n'est cependant donnée qu'à titre indicatif et qui n'a pas pour but de la limiter.

Selon la présente invention, la couche culotte, qui trouvera notamment son application dans le domaine de l'hygiène infantile et/ou médicale, formée au moins d'un film extérieur au moins imperméable aux liquides et d'un film intérieur perméable de confort, entre lesquels est disposé un matelas de matériau absorbant, la dite couche présentant en outre des moyens d'élastification de la ceinture ainsi que des moyens de fermeture de la couche culotte au niveau de la ceinture, est caractérisée par le fait que les dits moyens d'élastification et les dits moyens de fermeture sont formés par au moins une bande de matière élastique adhésive, disposée transversalement au niveau de la ceinture et définissant à ce niveau de part et d'autre de la couche culotte substantiellement des zones de fixation des dits moyens de fermeture, et qu'elle présente des zones adhésivées complémentaires, disposées au niveau de la ceinture, aptes à coopérer avec les dites zones de fixation pour assurer la fermeture et le maintien de la couche culotte.

La présente invention sera mieux comprise à la lecture de la description suivante, accompagnée des dessins en annexe qui en font partie intégrante.

Les figures 1a et 1b illustrent une couche culotte selon un premier mode de réalisation de la présente invention, montrant respectivement la face interne et la face externe de la couche avant son utilisation.

La figure 2 montre une vue en perspective d'une couche culotte, telle que représentée aux figures 1, formée pour son utilisation.

Les figures 3a et 3b illustrent une couche culotte selon un second mode de réalisation de la présente invention montrant respectivement la face interne et la face externe de la couche culotte avant son utilisation.

La figure 4 montre une vue en perspective d'une couche culotte, telle que représentée aux figures 2, formée pour son utilisation.

La figure 5 montre une vue partielle en coupe de la couche telle que représentée à la figure la pour mettre en évidence les détails de la ceinture.

La figure 6 montre une variante de la couche culotte telle que représentée à la figure 1.

La figure 7 montre une variante de réalisation de la ceinture d'une couche selon la présente invention.

L'invention vise une couche culotte, qui trouvera notamment son application dans le domaine de l'hygiène infantile et/ou médicale.

Les couches culottes et notamment les couches culottes dites jetables subissent constamment une évolution et sont perfectionnées pour, d'une part, abaisser leur coût de revient et d'autre part faciliter leur utilisation.

L'objet de la présente demande porte sur des perfectionnements apportés au niveau de la ceinture et des systèmes de fermeture dans le but d'augmenter l'étanchéité au niveau de la ceinture, de favoriser son maintien en place sur l'individu lors des mouvements et d'améliorer l'adaptation à l'anatomie du porteur.

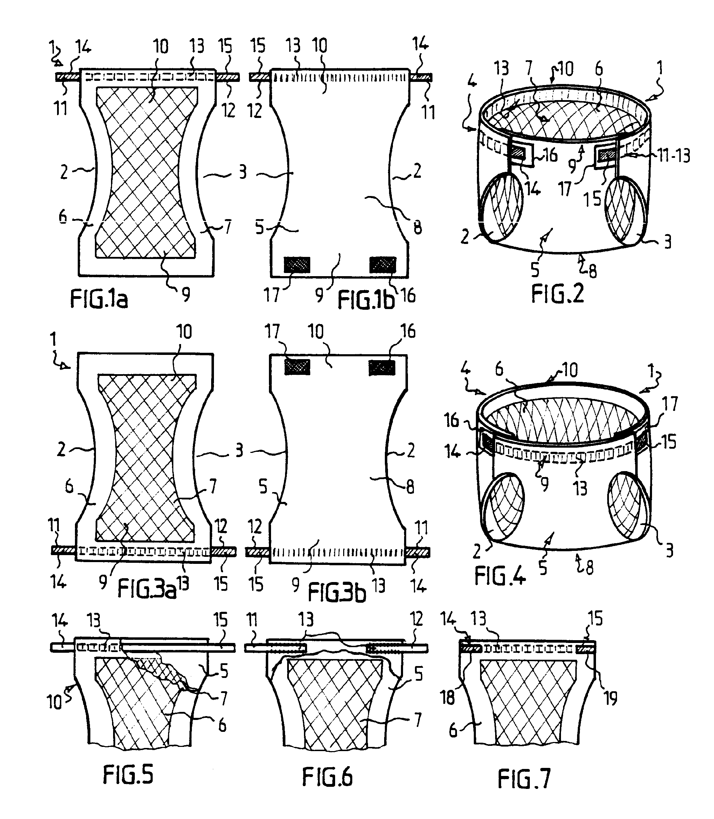

Comme le montrent les différentes figures 1 à 4, la couche culotte 1 est réalisée selon un type traditionnel et comporte des découpes latérales 2 et 3 pour permettre le passage des jambes et peut être pliée de telle façon qu'elle forme une ceinture 4 apte à s'adapter à la morphologie du porteur.

Pour ce qui est de la structure proprement dite, la couche culotte 1 est formée traditionnellement par une feuille extérieure 5, imperméable, étanche au moins au liquide, généralement réalisée par un film de polyéthylène, ainsi que par une feuille intérieure perméable de confort 6, destinée à s'appliquer vers l'utilisateur, réalisée généralement en voile de non tissé.

Entre la feuille extérieure imperméable 5 et la feuille intérieure perméable 6 est disposé un matelas central de matériau absorbant 7, généralement constitué d'ouate de cellulose ou tout autre matériau absorbant, voire même hyperabsorbant. A cet égard, pour former une enveloppe dans laquelle est renfermé le dit matériau absorbant, les feuilles extérieure 5 et intérieure 6 sont fixées mutuellement sur la périphérie la la couche culotte 1.

Ainsi formée, la couche culotte montre une partie centrale 8, présentant les dites découpes 2 et 3 pour constituer l'entrejambe, réunissant d'une part une partie avant 9 et d'autre part une partie arrière 10.

En outre, pour constituer la ceinture 4, la couche culotte 1 présente des moyens de fermeture 11, 12, prévus selon les cas au niveau des parties avant 9 ou arrière 10. Ces moyens seront décrits plus en détail ultérieurement.

Par ailleurs, pour l'étanchéité au niveau de la ceinture, la couche 1 de la présente invention comporte des moyens d'élastification 13 notamment prévus pour que la couche épouse parfaitement la taille du porteur et pour empêcher les remontées d'humidité.

Enfin, il est à noter que la couche culotte 1 de la présente invention pourrait être équipée d'autres perfectionnements qui sortent du cadre de la présente invention tel que par exemple, présence d'élastique au niveau des entrejambes ou autres.

Selon le perfectionnement de la présente invention, les dits moyens d'élastification 13 et les dits moyens de fermeture 11, 12 sont formés par au moins une bande de matière élastique adhésive disposée transversalement au niveau de la ceinture 4 et définissant à ce niveau de part et d'autre de la couche culotte 1 substantiellement des zones de fixation 14, 15 des dits moyens de fermeture 11, 12.

A ce sujet, il est à noter que ces zones de fixation 14, 15 et la ceinture élastique sont constituées par un seul et même matériau ce qui permet de répartir la contrainte non plus localement au niveau de la fixation de languettes sur la feuille imperméable, mais au contraire sur toute la longueur de la ceinture.

On évite ainsi les arrachements intempestifs de la feuille imperméable provoqués par la localisation des contraintes au niveau des dispositifs d'attache.

En outre, les dispositifs d'attache et la ceinture sont disposés dans le prolongement l'un de l'autre ce qui favorise la mise en place de la couche sur l'individu car la traction effectuée au niveau des zones de fixation 14, 15 au moment de la pose se répercute parfaitement dans l'alignement de la partie élastique 13 de la ceinture.

Dans un premier mode de réalisation, les zones de fixation 14, 15 des moyens de fermeture 11, 12 sont constituées par des languettes de fixation dépassant de part et d'autre des côtés latéraux de la couche au niveau de la ceinture, comme le montrent par exemple les figures 1 à 6.

Dans un second mode de réalisation, on pourrait envisager que ces languettes de fixation ne soient pas dépassantes et soient substantiellement constituées par des pavés 18, 19 disposés soit sur la feuille extérieure 5, soit sur la feulle intérieure 6 dans les parties latérales de la couche au niveau de la ceinture comme le montre par exemple la figure 7.

Cela étant, la couche culotte 1 présente, selon la présente invention, des zones adhésivées 16, 17, disposées au niveau de la ceinture 4, aptes à coopérer avec les dites zones de fixation 14, 15, quelle que soit leur réalisation, pour assurer la fermeture et le maintien de la couche culotte 1.

Selon un mode de réalisation avantageux de la présente invention, la dite bande élastique adhésive 11, 12, 13 et les dites zones adhésivées complémentaires 16, 17 se présentent sous la forme de deux substance adhésives complémentaires aptes à coopérer entre elles lorsqu'elles sont en vis-à-vis, la couche culotte étant en forme comme le montrent par exemple les figures 2 et 4.

Cela étant, la dite bande de matière élastique adhésive 11, 12, 13 se présente sous la forme d'un matériau extrudé élastomérique et auto-adhésif de type "hot melt" se collant sur lui-même à froid.

De même, pour coopérer avec la bande précitée, les dits pavés 16, 17 se présenteront avantageusement sous la forme d'une enduction d'adhésif "hot melt".

A titre d'exemple, la dite bande de matière élastique adhésive 11, 12, 13 est disposée, comme le montre particulièrement la figure 5, entre le dit film extérieur 5 et le dit film intérieur 6, soit à la partie arrière 10 comme le montrent les figures 1, soit à la partie avant 9 comme le montrent les figures 3.

Toutefois, il est également envisageable de disposer la bande 11-13 sur le film extérieur 5 et/ou sur le film intérieur 6.

Par ailleurs, dans le mode de réalisation de la figure 6, la matière constituant la partie 11 de la bande peut être déposée entre les deux films 5, 6 et un retour de matière peut être prévu soit sur le film extérieur 5, soit sur le film intérieur 6 pour constituer substantiellement les dits pavés de fixation 18-19.

Cela étant, cette bande est, soit placée à l'état tendu sur le film non plissé, soit dans un état non tendu sur le film plissé au moins partiellement.

A ce sujet, il est à noter que le polyéthylène constituant le film extérieur 5 a tendance à se froncer naturellement et il n'est par obligatoire de froncer de façon importante le film extérieur. Pour ce qui est du non-tissé, celui-ci possède une élasticité relative suffisante pour accepter l'élongation de la ceinture.

La dite bande extrudée de matière élastomérique présentera des dimensions variables selon l'utilisation et une largeur de ceinture de un à cinq centimètres sera requise. Par ailleurs, en ce qui concerne la densité de la matière, elle sera comprise entre cinquante et cinq cents grammes par mètre carré.

Ainsi, on réalise une ceinture présentant des capacités d'élasticité largement suffisantes pour l'utilisation couche culotte.

Pour ce qui est des zones adhésives 16, 17, elles se présentent sous la forme de pavés de colle, disposés de part et d'autre des zones latérales des parties avant 9 ou arrière 10 de la couche 1 selon que la ceinture est disposée en partie arrière 10 ou en partie avant 9 comme le montrent respectivement les figures 1 et 3.

De plus, ces pavés de colle 16, 17 sont disposés notamment sur la face externe du film extérieur 5 de façon à être en vis-à-vis des languettes de fixation 14, 15, comme le montrent les figures 2 et 4. Les matériaux étant complémentaires, on aura une adhésion des languettes sur les pavés de colle. De plus, la matière est telle que celle-ci autorisera un repositionnement des dispositifs de fixation.

On utilisera avantageusement comme précisé ci-dessus une enduction de matériau "hot melt" de même nature que le matériau constituant la bande 11, 12, 13. Toutefois, dans ce cas, la densité sera plus faible, de l'ordre de vingt à cent grammes par mètre carré, étant donné qu'on n'a pas besoin d'élasticité à ce niveau. En matière de dimensionnement de ces pavés, pour permettre un positionnement voire même un repositionnement des languettes de fixation 14, 15 sur ceux-ci, les pavés auront avantageusement par exemple une surface jusque dix fois supérieure à la surface des languettes.

Enfin, étant données les matériaux utilisés pour constituer la bande de matière élastique adhésive 11, 12, 13, en plaçant la ceinture entre le film extérieur 5 et le film intérieur 6, on réalisera la solidarisation de ces deux films. En outre, elle pourra être disposée le plus près possible du bord latéral de la partie avant 9 et/ou arrière 10 ; ainsi, on évitera tout effet de "bavette".

Cela étant, il est à noter que la bande de matière adhésive élastique 11, 12, 13 décrite jusqu'à présent de façon continue peut être également discontinue comme le montre par exemple la figure 6.

Naturellement, d'autres mises en oeuvre de la présente invention aurait pu être envisagées sans pour autant sortir du cadre de la présente invention.

## Revendications

1. Couche culotte (1), qui trouvera notamment son application dans le domaine de l'hygiène infantile et/ou médicale, la dite couche (1) étant formée au moins d'un film extérieur (5), au moins imperméable aux liquides, et d'un film intérieur (6), perméable de confort, entre lesquels est disposé un matelas (7) de matériau absorbant, la dite couche présentant en outre au niveau de la ceinture (4) des moyens d'élastification (13) de la ceinture ainsi que des moyens de fermeture (11, 12) de la couche culotte, lesdits moyens d'élastification (13) et lesdits moyens de fermeture (11, 12) étant formés par au moins une bande disposée transversalement au niveau de la ceinture (4) et définissant à ce niveau de part et d'autre de la couche culotte (11) substantiellement des zones de fixation (14, 15) des dits moyens de fermeture (11, 12), et que ladite couche présente des zones adhésives complémentaires (16, 17) disposées au niveau de la ceinture (4), aptes à coopérer avec les dites zones de fixation (14, 15) pour assurer la fermeture et le maintien de la couche caractérisée par le fait que la dite bande est une bande de matière à la fois élastique et adhésive (11, 12, 13) disposée, au niveau de la partie avant (9) ou arrière (10) de la couche intérieurement entre le film extérieur (5) et le dit film intérieur (6).

2. Couche culotte, selon la revendication 1, caractérisée par le fait que la dite bande élastique adhésive (11, 12, 13) et les dites zones adhésives complémentaires (16, 17) se présentent sous la forme de deux substances adhésives complémentaires aptes à coopérer entre elles lorsqu'elles sont en vis-à-vis, la couche culotte (1) étant en forme.

3. Couche culotte, selon la revendication 1, caractérisée par le fait que la dite bande (11, 12, 13) de matière élastique adhésive est placée à l'état tendu sur le film extérieur (5) non plissé.

4. Couche culotte, selon la revendication 1, caractérisée par le fait que la dite bande (11, 12, 13) de matière élastique adhésive est placée dans un état non tendu sur un film extérieur (5) plissé au moins partiellement.

5. Couche culotte, selon la revendication 1, caractérisée par le fait que la dite bande de matière élastique adhésive (11, 12, 13) se présente sous la forme d'un matériau extrudé élastomérique et auto-adhésif de type "hot melt" se collant sur lui-même à froid.

6. Couche culotte selon la revendication 1, caractérisée par le fait que la dite bande (11, 12, 13) a une densité de l'ordre de cinquante à cinq cents grammes par mètre carré.

7. Couche culotte, selon la revendication 1, caractérisée par le fait que les dites zones adhésives complémentaires (16, 17) se présentent sous la forme de pavés de colle, disposés de part et d'autre des zones latérales des parties avant (9) ou arrière (10) de la couche (1), sur la face externe du dit film extérieur (5).

8. Couche culotte, selon la revendication 7, caractérisée par le fait que les dits pavés de colle (16, 17) se présentent sous la forme d'une enduction de matériau "hot melt".

9. Couche culotte, selon la revendication 7, caractérisée par le fait que les les dits pavés de colle (16, 17) ont une densité de l'ordre de vingt à cent grammes par mètre carré.

## Patentansprüche

1. Windel (1) zur Anwendung insbesondere im Bereich der Kinderhygiene und/oder der medizinischen Hygiene, wobei diese Windel (1) aus mindestens einer äusseren Lage (5), welche zumindest für Flüssigkeiten undurchlässig ist und aus einer inneren durchlässigen und komfortablen Lage (6) aufgebaut ist, zwischen welchen ein Kissen (7) aus saugfähigem Material angeordnet ist, wobei die Windel ferner im Bereich des Gürtelbandes (4) Mittel (13) zur Elastifizierung des Gürtelbandes sowie Mittel zum Verschliessen (11, 12) der Windel aufweist, wobei die Mittel zur Elastifizierung (13) und die Mittel zum Verschliessen (11, 12) von zumindest einem Band gebildet sind, welches transversal im Bereich des Gürtelbandes (4) angeordnet ist und welches in diesem Bereich auf beiden Seiten der Windel (1) im wesentlichen Befestigungszonen (14, 15) für die Verschlussmittel (11, 12) bildet, und wobei die Windel im Bereich des Gürtelbandes (4) zusätzliche Klebezonen (16, 17) aufweist, die so ausgebildet sind, dass sie mit den Befestigungszonen (14, 15) zusammenarbeiten können, um die Verschliessung und das Festhalten der Windel zu garantieren, dadurch gekennzeichnet, dass dieses Band ein Band aus einem gleichzeitig elastischen und klebenden Material (11, 12, 13) ist, welches im Bereich der vorderen Partie (9) oder der hinteren Partie (10) der Windel im Inneren zwischen der äusseren Lage (5) und der inneren Lage (6) angeordnet ist.

2. Windel nach Anspruch 1, dadurch gekennzeichnet, dass dieses elastische und klebende Band (11, 12, 13) und die zusätzlichen Klebezonen (16, 17) in Form von zwei komplementären Klebesubstanzen vorliegen, die fähig sind miteinander zusammenzuarbeiten wenn sie sich gegenüber liegen wobei die Windel Ihre Form annimmt.

3. Windel nach Anspruch 1, dadurch gekennzeichnet, dass dieses Band (11, 12, 13) aus elastischem klebenden Material im gespannten Zustand auf die faltenlose äussere Lage (5) aufgebracht ist.

4. Windel nach Anspruch 1, dadurch gekennzeichnet, dass dieses Band (11, 12, 13) aus elastischem klebenden Material im ungespannten Zustand auf die äussere, zumindest teilweise in Falten liegende Lage (5) aufgebracht ist.

5. Windel nach Anspruch 1, dadurch gekennzeichnet, dass dieses Band (11, 12, 13) aus elastischem klebenden Material in Form eines extrudierten polymeren selbstklebenden Materials des Typs "hot melt", welches sich im kalten Zustand mit sich selbst verklebt, besteht.

6. Windel nach Anspruch 1, dadurch gekennzeichnet, dass dieses Band (11, 12, 13) eine Dichte im Bereich von 50 bis 100 g/m² aufweist.

7. Windel nach Anspruch 1, dadurch gekennzeichnet, dass die zusätzlichen Klebezonen (16,17) in Form von Kleberelementen vorliegen, welche auf beiden Seiten der Seitenbereiche der vorderen Partie (9) oder der hinteren Partie (10) der Windel (1) auf der Aussenfläche der äusseren Lage (5) aufgebracht sind.

8. Windel nach Anspruch 7, dadurch gekennzeichnet, dass die Kleberelemente (16, 17) in Form einer Beschichtung mit einem "hot melt" Material vorliegen.

9. Windel nach Anspruch 7, dadurch gekennzeichnet, dass die Kleberelemente (16, 17) eine Dichte im Bereich zwischen 20 und 100 g/m² aufweisen.

## Claims

1. A baby's napkin-pant (1), which is especially adapted for application in the field of infantile and/or hygiene, said napkin-pant (1) comprising at least one external, at least liquid tight film (5) and one internal, confort permeable, film (6), between which a mat (7) made of absorbing material is disposed, said mat further having past the belt (4) thereof means for elastifying (13) said belt as well as means for closing (11, 12) said napkin-pant, said elastifying means (13) and said closing means (11, 12) being formed of at least a strip transversally disposed at said belt (4) level and defining at said level on both sides of the napkin-pant (11) substantially regions (14, 15) for fixing said closing means (11, 12), and said napkin having complementary adhesive regions (16, 17) arranged at the belt (4) level which can cooperate with said fixing zones (14, 15) to allow said napkin to be positively closed and maintained, characterized in that said strip is a strip made of both resilient and adhesive material (11, 12, 13) inwardly arranged, at the front (9) or back (10) portion level of said napkin, between said external film (5) and internal film (6).

2. A baby's napkin-pant (1) as recited in claim 1, characterized in that said resilient adhesive strip (11, 12, 13) and said complementary adhesive regions (16, 17) are made of two complementary adhesive substances which can cooperate with one another when in a facing relationship, which said napkin-pant (1) being into position.

3. A baby's napkin-pant (1) as cited in claim 1, characterized in that said strip (11, 12, 13) made of resilient adhesive material is placed in a taut state on the non-pleated external film (5).

4. A baby's napkin-pant (1) as cited in claim 1, characterized in that said strip (11, 12, 13) made of resilient adhesive material is placed in a non-taut state on an at least partially pleated external film (5).

5. A baby's napkin-pant (1) as recited in claim 1, characterized in that said strip made of resilient adhesive material (11, 12, 13) comprises an elastomere and auto-adhesive extruded material of a "hot-melt" type which adheres on itself in a cold state.

6. A baby's napkin-pant (1) as recited in claim 1, characterized in that said strip (11, 12, 13) has a specific gravity from about fifty to five hundred grams/square meter.

7. A baby's napkin-pant (1) as recited in claim 1, characterized in that said complementary adhesive regions (16, 17) comprise glue slabs, disposed on both sides of lateral regions of said front (9) or back (10) portions of said napkin (1), on the external face of said external film (5).

8. A baby's napkin-pant (1) as recited in claim 7, characterized in that said glue slabs (16, 17) are obtained by coating with a "holt-melt" material.

9. A baby's napkin-pant (1) as recited in claim 7, characterized in that said glue slabs (16, 17) show a specific gravity from about twenty to hundred grams/square meter.
